# EUROPEAN PATENT APPLICATION

(11) **EP 1 878 445 A1**
(43) Date of publication of application: **16.01.2008**
(21) Application number: 06722367.7
(22) Date of filing: 19.04.2006
(51) Int. Cl.: A61K 47/10, A61K 47/14, A61K 47/20, A61K 47/26, A61K 47/32, A61K 47/44, A61K 31/7028, A61K 9/08, A61K 9/48, A61P 1/04, A61P 9/12, A61P 9/00, A61P 31/00, A61P 25/18, A61P 35/00, A61P 7/10, A61P 25/06, A61P 31/12, A61P 29/00, A61P 5/00

(54) **A METHOD, FORMULATION AND USE THEREOR WITH IMPROVED ORAL ABSORPTION OF DRUGS OR NUTRIENTS**

(30) Priority: 19.04.2005 CN 200510067126
(71) Applicant: Shanghai Tianbo Biotechnology Co., Ltd., Shanghai 200233 (CN)
(72) Inventor: HUANG, Dong, Surrey, BC V4N 4P8 (CA)
(74) Representative: Korga, Leokadia
(86) International application number: PCT/CN2006/000718
(87) International publication number: WO 2006/111085

(57) **Abstract**

This invention relates to the development of a composition formulation and method employed to alter the absorption site of orally administrated drugs, to promote the absorption of bioactive lipophilic and/or hydrophilic compounds from the gastrointestinal tract, and as a result to increase drug bioavailability. Applications of the methods and formulations according to the invention are also described. The composition formulation comprises pharmaceuticals or nutraceuticals (hydrophilic and/or lipophilic compounds) in combination with one or more organic solvents and one or more acid protectants. The composition formulation is formulated into a dosage form, which is in conformity with pharmaceutical or nutraceutical requirements, is administrated orally to facilitate the absorption, and can avoid undesired degradation and metabolism of pharmaceuticals and nutraceuticals in the gastrointestinal tract thereby increasing their overall bioavailability.

## Description

Field of invention

This invention relates to the development of a composition formulation and a method employed to change the absorption site of orally administrated drugs, and promote the absorption of bioactive lipophilic and/or hydrophilic compounds from the gastrointestinal tract thereby increasing drug bioavailability. Applications of the methods and formulations according to this invention are also described.

Background of the invention

Oral administration is the most commonly used and convenient route of administration of pharmaceuticals and nutrients that enter blood stream though the gastrointestinal tract via various absorption modalities. However, due to the hydrophilic and/or lipophilic properties of pharmaceuticals and nutraceuticals developed from natural plants, the absorption of oral formulations through the gastrointestinal tract is influenced by many factors which include intrinsic properties (e.g., individual absorption mechanism, chemical and physical properties, solubility, and dissolving speed), and extrinsic GI factors (e.g., pH value, gastric emptying speed, intestinal peristalsis, gastric contents, and bacterial flora). Therefore, the overall absorption and bioavailability are compromised to a degree that their practical application and pharmacological efficacy are restricted.

In general, the gastrointestinal (GI) tract is the main absorption site for oral pharmaceuticals and nutraceuticals. Within the Gl tract, the intestinal tract is generally regarded as the main absorption site in view of the large absorption area of the intestines and the fast passage of drugs through the stomach. When taken orally, drugs will disintegrate in the stomach and further undergo degradation, enzymolysis, or precipitation in the gastric environment under the influence of the low pH value, bioenzymes, and bacterial flora. Furthermore, drugs pass through the stomach so quickly (less than 2 hours) that only a small portion is absorbed therein. Meanwhile, the absorption differs between hydrophilic and lipophilic drugs.

Lipophilic pharmaceuticals or nutraceuticals are soluble in fats (oil) and organic solvents, but generally not soluble or sparingly soluble in water. The low solubility of lipophilic bioactive compounds in water is one of the important limiting factors to their therapeutic application in patients. Even when administrated orally in an oil solution, special water solution, oil suspension, or emulsion, their absorption rate and bioavailability are still low. Lipophilic bioactive compounds are easy to precipitate under the action of gastric acid and water. This type of precipitated form is not otherwise absorbable. Although the hydrophilic bioactive chemicals can be dissolved in either water or a majority of organic solvents and not precipitated in stomach, these drugs are vulnerable to degradation/hydrolysis, and enzymolysis to become inactive metabolites under the action of acids, water, various enzymes, and bacteria. Accordingly, only a small proportion of hydrophilic drugs in their original forms are absorbed and enter the blood stream. As a result, the pharmacological activity of original drugs cannot be satisfactorily reached.

The challenges described above also exist in the realm of pharmaceuticals and nutraceuticals developed from natural plants. A fairly large portion of natural plant derived pharmaceuticals and nutraceuticals belong to the category of saponins. Due to their therapeutic and nutraceutical values, saponins such as ginsenosides, notogenseng saponins, and other damarane saponins have been commercialized and used clinically to prevent and to treat a variety of diseases, or extensively as nutrition supplements. Some water soluble saponins are hydrolyzed and/or enzymolyzed (their chemical structure is modified) in stomach by gastric acid as well as in intestinal tract by enzymes. Only an extremely small amount of saponins in their intact forms can be metabolized by intestinal bacterial flora into active forms and subsequently absorbed into blood. Nevertheless, the lipophilic saponins will form precipitates in gastric and intestinal environments, and as a result, they are almost not at all absorbed in the stomach and the intestines.

For example, panoxadiol and panoxatriol saponins (ginsenosides), the major pharmacologically active ingredients of ginseng, are hydrophilic compounds having a very low absorption rate from the GI tract, with the absorption rate being only 1 % for Rb1, 3.4% for Rh2, and 1.9% for Rg1 (Dong, SH, et. al, In vivo metabolism Studies of ginsenoside, Renshen Yanjiu. 2003,Vo1. 15 (1) 2-6). It was also reported by Wang that, after being administered orally to mice, ginsenoside Rg1 is degraded into Rh1, and C-25 hydroxy Rh1, and Rb1 and Rb2 are also transformed into hydroxylated forms of C-25 and C-24, and peroxidates of C-25 and C-24 (Wang, LP, et. al, In vivo metabolism of ginsenoside, Strait Pharmaceutical Journal. 2000, Vol.12, (4)4-6). In addition, Karikura et al found that ginsenoside Rh2 is hydrolyzed in the stomach into 4 forms: 25-OH-23-alkene, 24-OH-25-alkene, a derivative of 25-hydroperoxyl-23-alkene, and a derivative of 24-hydroperoxyl-25-alkene. (Karikura et al, Chem. Pharm. Bull. (Tokyo) 1999 Feb, 39 (2):400*)*

During the absorption process of hydrophilic ginsenosides, the original ginsenosides are hydrolyzed, degraded, and metabolized into secondary or terminal metabolites which are absorbed subsequently. Therefore, the biological and pharmacological activities of ginsenosides present in their original forms are greatly compromised, and their pharmaceutical value is lowered correspondingly.

Alkaloids and flavanoids are also the main ingredients extracted from natural plants. With their high pharmaceutical and nutraceutical values, alkaloids and flavanoids, such as soy isoflavone, genistein, bilobalide, propolis are also used as drugs or nutrition supplements for disease prevention and treatment. Alkaloids and flavanoids are also classified into hydrophilic and lipophilic compounds, and encounter the same absorption problems as ginsenosides.

In order to avoid the influence of gastric and intestinal environments on the pharmacological activity of pharmaceuticals and nutraceuticals, many efforts and extensive research have been made in the pharmaceutical sector to prevent drug decomposition in the stomach, and to accomplish a controlled disintegration, release, and absorption of drugs in the Gl. Specifically, drugs can be coated with or enclosed by materials undegradable by gastric fluid to prevent drug disintegration and release in the stomach, and control the drug release in the intestines so as to achieve desirable blood concentration and bioavailability of drugs and good therapeutic effects consequently. Even though a fair amount of drugs reach the intestines without having undergone gastric destruction, the absorption rate and bioavailability of these drugs are greatly dictated by their chemical and physical natures as well as the intestinal environment (pH value, enzymes, bacteria etc.), and are not satisfying.

From the perspective of practicality, it is imperative to develop a method which can protect original pharmaceuticals and nutraceuticals from gastric degradation and destruction by acids, enzymes, and bacteria, and also prevent their precipitation in the stomach, as well, shift the drug absorption away from the intestine and other influencing factors, so as to increase the absorption proportion of original pharmaceuticals or nutraceuticals in the stomach without undesired metabolism, and augment the overall absorption rate and the bioavailability, consequently.

Description of the invention

The invention aims to overcome the shortcomings of current oral administration technologies by developing a method and formulation to change the absorption site of oral pharmaceuticals and nutraceuticals and improve their overall bioavailability. By avoiding any absorption-influencing factors, the proposed method and formulation will effectively protect pharmaceuticals and nutraceuticals from degradation in the gastric environment, and keep them in an intact form, a dissolved form, or a molecular state ready for absorption, thereby inverting the absorption profile of drugs in stomach versus that in the intestines. The method increases the stability of therapeutically effective pharmaceuticals and nutraceuticals when subjected to gastric and intestinal environments, and prevents the hydrolysis, destruction, and precipitation in the GI tract. Meanwhile, this method alters the conventional absorption site of pharmaceuticals and nutraceuticals, i.e., the intestines, to the stomach, and greatly improves the overall absorption rate and bioavailability.

The method and composition formulation provided by this invention can be used for and is applicable to oral dosage form preparations of hydrophilic and/or lipophilic chemical compounds used for pharmaceutical and nutraceutical purposes. Moreover, formulations of this invention protect the mixed compounds from degradation, destruction, and precipitation in the GI tract, improve the compounds' absorption in the stomach, and alter the conventional absorption site of pharmaceuticals and nutraceuticals, i.e., the intestines, into the stomach, greatly improving the overall absorption rate and bioavailability.

In order to materialize the embodiments of the invention, a formulation of organic solvents is utilized to produce the oral dosage forms of hydrophilic, lipophilic, and/or mixed compounds for pharmaceutical or nutraceutical purposes. The oral dosage forms developed by this method can resist the influence of acids, water, enzymes, and bacteria in the stomach, and avoid their precipitation, thereby improving the drug absorption in the stomach in their intact forms rather than their metabolite forms, and greatly increase the overall absorption rate and bioavailability of pharmaceuticals and nutraceuticals as well as their biological and pharmacological activities.

The formulation used in embodiments of this invention comprises a mixture of hydrophilic and/or lipophilic pharmaceuticals and nutraceuticals; with one or more of the following organic solvents, including but not limited to, fish oil, cardy oil, refined soybean oil, refined peanut oil, eveninig primrose oil, Silybum marianum Gaertn oil, grape seed oil, sunflower seed oil, Seabuckthorn fruit oil, other vegetable oils, and ethanol, propylene glycol, glycerin, polyethylene glycol, ethyl acetate, and propylene carbonate; and one or more of the following acid protectants, including but not limited to, Labrafil M 1944 CS, tween 20, tween 21, tween 40, tween 60, tween 80, tween 81, Brij, Spans, polyethylene glycol 200, polyethylene glycol 600, soybean phospholipids, lecithin, polyglyceride, sucrose esters, polyoxyethylene hydrogenated vegetable oil (e.g., polyoxyethylene hydrogenated castor oil /Cremophor RH40), polyoxyethylene-anhydro-sorbitol fatty acid esters (e.g., polyoxyethylene- anhydro-sorbitol monolaurate, tween 20), Nikkol HCO 40, Nikkol HCO 50, Nikkol HCO 60, sodium dodecylsulfate, magnesium dodecylsulfate, sodium dodecylsulfonate, sodium tetradecyl sulfate, and glyceryl monostearate(s).

The above formulation is made into oral dosage forms such as solution, a liquid capsule, or a soft capsule.

The optimized methods and compositions of the invention comprise hydrophilic and/or lipophilic pharmaceuticals or nutraceuticals, ethanol of various concentrations, and polyoxyethylenes of hydrogenated vegetable oils (polyoxyethylene hydrogenated caster oil / Cremophor RH40) of various proportions. The above formulation is made into well-recognized oral dosage forms including but not limited to oral solution, soft capsules, and hard gelatin liquid capsules. The pharmaceuticals and nutraceuticals in the formulation are compatible at least with ethanol and polyoxyethylene hydrogenated vegetable oils (polyoxyethylene hydrogenated caster oil / Cremophor RH40).

Other pharmaceutically acceptable pharmaceutical vehicles or adjuvants including solubilizing agents, surfactants, and food additives can also be added.

In certain embodiments, the invention features the following:
1) Ethanol (or other above-mentioned organic solvents) at various concentrations and polyoxyethylene hydrogenated caster oil (or other above-mentioned acid protectants) of various proportions are utilized in the formulation to protect the dissolved drugs from decomposition, metabolism, and precipitation in the gastric and intestinal environments (acids, enzyme, bacteria etc.).
2) The organic solvents of this formulation can dissolve not only the hydrophilic or lipophilic bioactive compounds, but also the mixture thereof.
3) The composition formulation of the invention features gastric absorption of active components of drugs, and changes the conventional absorption site from the intestines to the stomach.
4) The formulation developed in embodiments of the invention can effectively prevent the degradation and metabolism of drug components, keep original drugs in their intact forms having biological and pharmacological activities, and avoid potential adverse effects of their secondary metabolites.

In certain embodiments of the invention, the method comprises the following steps:
1) Determining the solubility of pharmaceuticals and/or nutraceuticals in organic solvents. The solubility of pharmaceuticals and nutraceuticals in organic solvents is determined using well-recognized methodologies, or is obtained from the literature. The proportions of components in the formulation are calculated according to the solubility of pharmaceuticals and nutraceuticals in organic solvents.
2) (1). Completely dissolving pharmaceuticals or nutraceuticals in organic solvents of various concentrations to obtain an "intermediate solution A". The organic solvents are one or more than one of the followings: fish oil, cardy oil, refined soybean oil, refined peanut oil, eveninig primrose oil, Silybum marianum Gaertn oil, grape seed oil, sunflower seed oil, Seabuckthorn fruit oil, other vegetable oils, and ethynol, propylene glycol, glycerin, polyethylene glycol (PEG), ethyl acetate, and propylene carbonate; (2). Determining the acid protectant proportion in the "intermediate solution A" using well-recognized methodologies. Acid protectants are selected from, but not limited to: Labrafil M 1944 CS, tween 20, tween 21, tween 40, tween 60, tween 80, tween 81, Brij, Spans, polyethylene glycol 200-polyethylene glycol 600, soybean phospholipids, lecithin, polyglyceride, sucrose esters, polyoxyethylene hydrogenated vegetable oil (e.g., polyoxyethylene hydrogenated castor oil / Cremophor RH40, polyoxyethylene-anhydro-sorbitol fatty acid esters (e.g., polyoxyethylene- anhydro-sorbitol monolaurate, tween 20), Nikkol HCO 40, Nikkol HCO 50, Nikkol HCO 60, sodium dodecylsulfate, magnesium dodecylsulfate, sodium dodecylsulfonate, sodium tetradecyl sulfate, and glyceryl monostearate(s). Thecomposition formulation added with acid protectants is mixed with artificial gastric or intestinal fluids to test its stability. Using complete dissolution without crystal precipitation as a standard, the proportion of acid protectants in formulation is determined;
3) In conformity with pharmaceutical and food requirements, the above-determined composition formulation is prepared into different well-recognized oral dosage forms, such as solution, soft capsule, liquid capsule. Other flavorings, edible pharmaceutical adjuvants, or food additives can be added, if needed.

The composition formulation of the invention comprises in weight percentages with respect to the entire formulation:
1) Pharmaceuticals or nutraceuticals: 5%-90%; optimally: 10%-50%.
2) Organic solvents (e.g., fish oil, cardy oil, refined soybean oil, refined peanut oil, eveninig primrose oil, Silybum marianum Gaertn oil, grape seed oil, sunflower seed oil, Seabuckthorn fruit oil, other vegetable oils, and ethynol, propylene glycol, glycerin, polyethylene glycol, ethyl acetate, and propylene carbonate): 5%-90%; optimally: 5%-50%.
3) Acid protectants (e.g., Labrafil M 1944 CS, tween 20, tween 21, tween 40, tween 60, tween 80, tween 81, Brij, Spans, polyethylene glycol 200-polyethylene glycol 600, soybean phospholipids, lecithin, polyglyceride, sucrose esters, polyoxyethylene hydrogenated vegetable oil (such as, e.g., polyoxyethylene hydrogenated castor oil / Cremophor RH40, polyoxyethylene-anhydro-sorbitol), fatty acid esters (such as, e.g., polyoxyethylene- anhydro-sorbitol monolaurate, tween 20), Nikkol HCO 40, Nikkol HCO 50, Nikkol HCO 60, sodium dodecylsulfate, magnesium dodecylsulfate, sodium dodecylsulfonate, sodium tetradecyl sulfate, and glyceryl monostearate(s)): 5%-90%; optimally: 12.5%-60%.

The composition formulation of the invention comprises:
1) Drug content: 5%-90% (by weight); optimally, 10%-50%.
2) Content of organic solvent (ethanol) 5%-90%; optimally, 5%-50%.
3) Content of acid protectants (polyoxyethylene hydrogenated castor oil / Cremophor RH40): 5%-90%; optimally, 12.5%-60%.

The method and composition formulation developed in embodiments of this invention can be used for the oral dosage form preparation of hydrophilic, and/or lipophilic bioactive compounds, as drugs, nutraceuticals, health products and foods, or functional foods.

The method and composition formulation described herein are embodied in several oral dosage forms including solution, soft capsule, hard gelatin capsule, etc. The compositions in these oral dosage forms are as described above.

The pharmaceuticals or nutraceuticals used in the embodiments of the invention refer to the hydrophilic, lipophilic bioactive compounds, or the mixture thereof. The optimized compositions are prepared from coumarins, triterpenoid saponins, steroid saponins, flavonoids, diterpene, triterpenoid lactone or quinones of natural products. The compositions comprise without limitation one or more of: ginsenosides Rh2 and Rg3, protopanoxadiol sapogenin, protopanoxatriol sapogenin, total ginsenosides, Radix Astragali saponins, platycodin, saikosides, dioscin, baicalin, kakkonein (puerarin), genistein, soybean isoflavone, bilobalide, propolis, psoralen, ammidin, salviol, etc.

Other well-recognized pharmaceutical vehicles and adjuvants including sweeteners, solubilizing agents, and surfactants can also be supplemented into the formulation, but are not indispensable components.

According to their biological activities and pharmaceutical properties, the invention makes possible a practical application of hydrophilic and lipophilic bioactive compounds in oral routes of administration for the prevention and treatment of peptic ulcer, pain, hypertension, viral and bacterial infection, parasite infection, inflammation, psychosis (sedatives), depression, cardiovascular diseases, tumors, muscarine intoxication, migraine, diabetes, aging, epilepsies, dyslipidemia, allergies (hydryllins), water retention (diuretics) etc.

Figure Legends

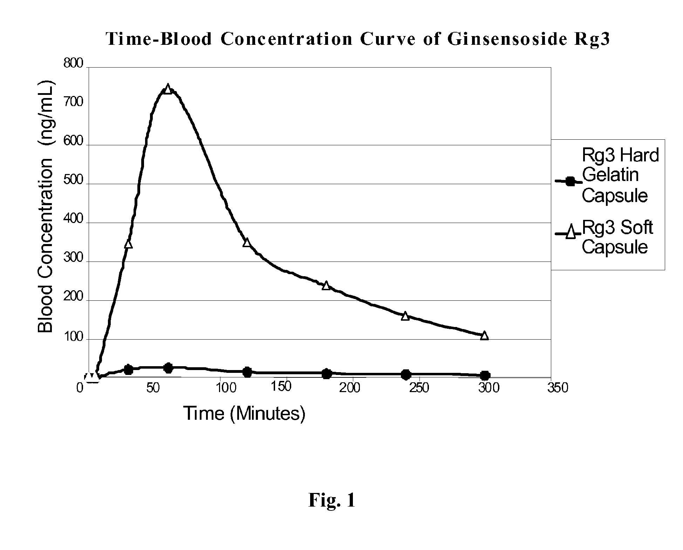

Fig. 1 is a time-blood concentration curve of ginsenosides Rg3 of various oral dosage forms; and

Fig. 2 is a time-blood concentration curve of protopanoxadiol aPPD of various dosage forms (injection versus oral administration).

Experimental Examples

As demonstrated in the appended figures, the invention is further described hereinafter in more detail. The examples are not meant to restrict the scope of the invention.

Example 1. Determination of solubility of hydrophilic and lipophilic bioactive compounds in ethanol

As defined in Chinese Pharmacopeia (version 2000), solubility can be classified into 7 categories: extremely soluble, easily soluble, soluble, sparingly soluble, slightly soluble, very slightly soluble, and almost insoluble or insoluble, each category is defined below:
Extremely soluble: > 1g (ml) of solute can be dissolved in 1 ml of solvent.
Easily soluble: 1g (ml) of solute can be dissolved in 1-10 ml of solvent.
Soluble: 1g (ml) of solute can be dissolved in 10-30 ml of solvent.
Sparingly soluble: 1g (ml) of solute can be dissolved in 30-100 ml of solvent.
Slightly soluble: 1g (ml) of solute can be dissolved in 100-1000 ml of solvent.
Very slightly soluble: 1g (ml) of solvent can be dissolved in 1000-10000 ml of solvent
Almost insoluble or insoluble: 1g (ml) of solute cannot be dissolved in 1000 ml of solvent.

A standard method for determining solubility is also defined in Chinese Pharmacopeia (version 2000): A certain amount of ground sample or solution the solubility of which is to be tested is added to a certain volume of organic solvent at 25°C±2°C, and forcefully shaken for 30 seconds every 5 minutes. The solution is observed at 30 minutes, and complete dissolution occurs when no solute particles or droplets are noticed.

Example 2. Imitation of gastric and intestinal environments.

Preparation of artificial gastric or intestinal fluids conforming to the appendix of capsules in "The General Principle of Pharmaceutical Preparation":
1) Artificial gastric fluid: mixed diluted hydrochloric acid and 10 g of pepsin with 800 ml of water; then add water into the mixture to 1000 ml of the final volume.
2) Artificial intestinal fluid: dissolve 6.8 g of dihydrogen phosphate in 500 ml of water; adjust the pH value to 6.8 using 0.4 % sodium hydroxide; prepare a solution of 10 g trypsin in water; then combine the trypsin solution with dihydrogen phosphate solution, and adjust the volume to 1000 ml.

Example 3. Preparation of oral dosage formulations of hydrophilic glycosides.
1) Dissolve ginsenoside Rg3 in ethanol.
   100 mg test ginsenoside Rg3 was added to 100 ml of 90% ethanol at 25°C±2°C, and forcefully shaken for 30 seconds every 5 minutes. The solubility was observed at 30 minutes, and it was ascertained that 100 mg Rg3 can be completely dissolved in 100 ml 90% ethanol.
2) Prepare Rg3 solution in ethanol.
   The ginsenoside Rg3-ethanol solution was prepared according to Rg3's solubility, and this solution was used to determine the percentage of polyoxyethylene hydrogenated castor oil in formulation.
3) Prepare artificial gastric and intestinal environments.
   The artificial gastric and intestinal fluids were prepared in accordance with experimental example 2.
4) Determine the proportion of protectant polyoxyethylene hydrogenated castor oil (Cremophor RH40) in ginsenoside Rg3-ethanol solution.
   Polyoxyethylene hydrogenated castor oil (Cremophor RH40) at different proportions, from 5%-90%, was added to 100 ml ginsenoside Rg3-ethanol solution, and then the mixture was subjected to artificial gastric or intestinal fluids for stability test. The optimal proportion of polyoxyethylene hydrogenated castor oil (Cremophor RH40) in Rg3-ethanol solution was obtained when the test drugs were not hydrolyzed, not metabolized, and not precipitated in gastric or intestinal fluid. The highest proportion as determined, i.e., 50 % or 175 mg was adopted for drug preparation.
5) Using above-obtained proportion of polyoxyethylene hydrogenated castor oil (Cremophor RH40), the oral dosage forms of ginsenoside Rg3 were prepared according to pharmaceutical and food standards and requirements.

Example 4. Preparation of oral dosage formulations of lipophilic glycosides.
1) Dissolve protonaxodiol sapogenin aPPD in ethanol.
   40 mg protopanoxadiol sapogenin aPPD was added into 90% ethanol at 25°C±2°C, and forcefully shaken for 30 seconds every 5 minutes. The solubility was determined at 30 minutes when no solute is observed in solution. 40 mg aPPD could be completely dissolved in 100 ml 90% ethanol.
2) Prepare aPPD solution in ethanol.
   100 ml of aPPD solution in ethanol is prepared according to above-obtained aPPD solutibility example, this solution is then used to determine the percentage of polyoxyethylene hydrogenated castor oil (Cremophor RH40) that should be used.
3) Prepare artificial gastric and intestinal environments.
   The artificial gastric and intestinal fluids are prepared in accordance with experimental example 2.
4) Determine the proportion of polyoxyethylene hydrogenated castor oil (Cremophor RH40) in aPPD-ethanol solution.
   Polyoxyethylene hydrogenated castor oil (Cremophor RH40) at different proportions of 5%-90% was added into 100ml aPPD-ethanol solution, and then the mixture was subject to artificial gastric or intestinal fluid for stability test. The optimal proportion of polyoxyethylene hydrogenated castor oil (Cremophor RH40) in Rg3-ethanol solution was obtained when the test drugs were not hydrolyzed, not metabolized, and not precipitated in gastric or intestinal fluid. The highest proportion as determined, i.e., 40% or 150 mg was adopted for drug preparation.
5) Using the above-obtained proportion of polyoxyethylene hydrogenated castor oil (Cremophor RH40), the oral dosage forms of aPPD were prepared in line with pharmaceutical and food standards and requirements.

Example 5. Preparation of oral dosage formulations of hydrophilic ginsenoside and lipophilic sapoginin mixtures.
1) Dissolve saponin and sapogenin mixture in ethanol.
   Weighed 150 mg saponin and sapogenin mixture, and put it into 100 ml 90% ethanol at 25°C±2°C, and forcefully shook it for 30 seconds every 5 minutes. The solubility was observed at 30 minutes. Result showed that 150 mg saponin and sapogenin can be completely dissolved in 100 ml 90% ethanol.
2) Prepare saponin-sapogenin-ethanol solution.
   According to above solubility of saponin and sapogenin mixture in ethanol, 100 ml drug-ethanol solution was prepared and used to determine the proportion of polyoxyethylene hydrogenated castor oil (Cremophor RH40).
3) Prepare artificial gastric and intestinal environments.
   The artificial gastric and intestinal fluids were prepared in accordance with experimental example 2.
4) Determine of the proportion of polyoxyethylene hydrogenated castor oil (Cremophor RH40) in saponin-sapogenin-ethanol solution
   Polyoxyethylene hydrogenated castor oil (Cremophor RH40) at different proportions of 5%-90% was added to 100 ml saponin-sapogenin-ethanol solution, and then the mixture was subjected to artificial gastric or intestinal fluid for stability testing. The optimal proportion of polyoxyethylene hydrogenated castor oil (Cremophor RH40) in saponin-sapogenin-ethanol solution was determined at a point when the test drugs were not hydrolyzed, not metabolized, and not precipitated in gastric or intestinal fluids. The highest proportion as determined, i.e., 37% or 150 mg, was adopted for drug preparation.
5) Using above-obtained proportion of polyoxyethylene hydrogenated castor oil (Cremophor RH40), the oral dosage forms of saponin-sapogenin mixture were prepared in line with pharmaceutical and food standards and requirements.

Example 6. Preparation of health products of bioactive lipophilic ginseng sapogenins in oral dosage form.
1) Dissolved 100 mg protopanoxadiol sapogenin aPPD in 120 mg ethanol, and mixed until complete dissolution.
2) Weighed 175mg polyoxyethylene hydrogenated castor oil (Cremophor RH40), placed it into vacuum emulsifying mixer, preheated the mixer to 35°C, then added above aPPD-ethanol solution into the mixer, and blended the mixture completely, the mixture looked clear and transparent.
3) Placed the above clear mixture into homogenizer to remove bubbles (defoaming), and then transferred it into a clean container.
4) According to pharmaceutical standards and requirements such as quality control and hygiene requirement, the above defoamed mixture was made into oral dosage forms.

Example 7. Measurement of bioavailabilities of saponin and sapogenin drugs

The bioavailabilities of invented dosage forms using the composition formulation and conventional dosage form were measured and compared. The results show that the invention can greatly improve the bioavailability of orally administrated drugs.
1) The blood concentration and bioavailability of ginsenoside Rg3 in a human.
(1) Measurement of blood drug concentration in a human.
(a) Target Population: 6 healthy adult volunteers age 40-45 years.
(b) Dosage forms for test: The composition formulation developed in embodiments of this invention was formulated into soft capsules. The composition formulation comprises: 100mg ginsenoside Rg3, 75 mg absolute ethanol, 175 mg polyoxyethylene hydrogenated castor oil (Cremophor RH40). The commercially-available hard gelation capsules of Rg3 (ShenYi Capsule) were used for comparison.
(c) Dosage: 300 mg, single dose.
(d) Blood Sample Collection: Blood samples were collected at 30, 60, 120, 180, 240, and 300 minutes after drug administration.
(e) Blood drug concentrations were measured.

(2) Blood drug concentrations are illustrated in Table 1.
(3) The relative bioavailabilities were compared.

| Time (Minutes) | Blood Concentration (ng/ml) | |
|---|---|---|
| | Rg3 Soft Capsule | Rg3 Hard Gelatin Capsule |
| 0 | | |
| 5 | | |
| 30 | 345±31 | 21±7 |
| 60 | 745±65 | 27±11 |
| 120 | 349.5±32 | 15±5 |
| 180 | 236.25±19 | 11±4 |
| 240 | 159.75±20 | 9±3 |
| 300 | 107.625±17 | 8±1 |

| Dosage Form | Parameter of Bioavailability (AUC) | Bioavailability Comparison(%) |
|---|---|---|
| | AUC₀₋₂₄/ng.h/ml | |
| Rg3 soft capsule | 1989.37 | 1722 % |
| Rg3 Hard gelatin Capsule | 115.5 | 1 |

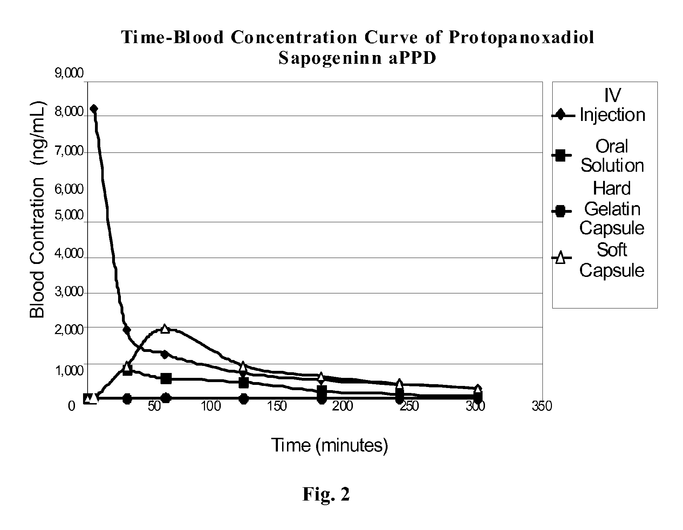

2) The blood concentration and bioavailability of protopanoxadiol drugs in human. The blood concentrations and bioavailabilities of oral soft capsule and injection solution developed in embodiments of this invention and hard gelatin capsule prepared using the conventional method were measured and compared. The results proved that the dosage forms developed by this invention can greatly increase the drug's bioavailability.
(1) Measurement of blood drug concentration in human:
(a) Target population: 12 healthy adult volunteers age 40-45
(b) Drugs:
   (i)Soft Capsule: Soft capsules for test are made using well-recognized method and the composition formulation developed in embodiments of this invention. The composition formulation comprises: 100 mg protopanoxadiol sapogenin aPPD, 120 mg absolute ethanol, and 150 mg polyoxyethylene hydrogenated castor oil (Cremophor RH40).
   (ii)IV Injection: The injection solution of protopanoxadiol sapogenin aPPD was prepared by the method developed in embodiments of this invention.
   (iii)Hard Gelatin Powder Capsule: The hard gelatin capsules of protopanoxadiol sapogenin aPPD were prepared by a well-recognized conventional method.
(c) Dosage: 800 mg, single dose.
(d) Blood Sample Collection: Blood samples were collected at 5 minutes before intravenous injection of aPPD, then once every 60 minutes after injection until 240 minutes. Blood samples were collected at 30, 60, 120, 180, 240, and 300 minutes after the oral capsules were taken.
(e) Blood drug concentrations were measured, averaged, and plotted in Figure 2.

(2) The absolute bioavailabilities of each dosage form are tabulated below:

| Time (minute) | Blood Concentration (ng/ml) | | | |
|---|---|---|---|---|
| | Injection | Oral Soft Capsule | Oral Solution | Oral Hard Gelatin Capsule |
| 0 | | | | |
| 5 | 8206±425 | | | |
| 30 | 1928±234 | 920±87 | 820±79 | 17±20 |
| 60 | 1266±137 | 1987±201 | 578±64 | 11±20 |
| 120 | 725±93 | 932±83 | 470±57 | |
| 180 | 534±39 | 630±57 | 210±19 | |
| 240 | 408±51 | 426±53 | 125±20 | |
| 300 | 281±19 | 287±20 | 83±13 | |

| Dosage Form | Bioavailability(AUC) | Bioavailability Comparison(%) |
|---|---|---|
| | AUC₀₋₂₄/ng.h/ml | |
| Injection(IV) | 6451 | 1 |
| Oral Soft Capsule | 5305 | 82.2% |
| Oral Solution | 2580 | 40.1 % |
| Oral Hard Gelatin Capsule | - | <1 % |

As demonstrated in above experiments, the bioavailability of hydrophilic ginsenoside Rg3 and lipophilic protopanoxadiol sapogenin aPPD in dosage forms developed by this invention is superior to that in hard gelatin powder capsule prepared using conventional methods. Hard gelatin capsule made by conventional methods is prone to disintegration, precipitation, or hydrolysis in gastrointestinal environment due to its physico-chemical properties, thereby resulting into a low absorption rate. By using protectants which protect the dissolved drugs from degradation in the gastrointestinal environment, and ethanol as absorption vehicle in the gastrointestinal tract, this invention allows for drugs to be kept dissolved in their molecular state ready for gastrointestinal absorption, shifts the conventional absorption site of ginsenosides and sapogenins from the intestines to the stomach, and greatly improves bioavailability.

## Claims

1. A pharmaceutical or nutraceutical composition formulation comprising:
a mixture of a pharmaceutical or an nutraceutical active drug component and at least one organic solvent; and
a protectant that can prevent decomposition of the component in a gastrointestinal environment.

2. The formulation of claim 1, wherein said organic solvent is selected from one or more of the following: fish oil, safflower oil, refined soybean oil, refined peanut oil, evening primrose oil, milk thistle oil, grape seed oil, sunflower oil, seabuckthorn oil, and other animal or plant oil, ethanol, propylene glycol, glycerol, polyethlene glycol (PEG), ethyl acetate, or propylene carbonate.

3. The formulation of claim 1, wherein said protectant is selected from one or more of the following: Labrafil M 1944 CS, Tween 20, Tween 21, Tween 40, Tween 60, Tween 80, Tween 81, Brij, Spans (sorbitan fatty acid ester), polyethlene glycol 200, polyethlene glycol 600, a soybean phospholipid, lecithin, a polyglycide, a sucrose ester, a polyoxyl hydrogenated vegetable oil, Cremophor RH 40, polyoxyethylene sorbitan fatty acid ester, POE(20), Nikkol HCO 40, Nikkol HCO 50, Nikkol HCO 60, sodium dodecyl sulphate, magnesium dodecyl sulphate, sodium dodecylsulfonate, sodium tetradecyl sulphate, or glycerol monostearate.

4. The formulation of claim 1, wherein said protectant is a polyoxyl hydrogenated castor oil, Cremophor RH 40.

5. The formulation of claim 4, wherein the weight proportions of said pharmaceutical or nutraceutical, said ethanol, and said polyoxyl hydrogenated castor oil, Cremophor RH 40, with respect to the formulation are in the range of 5%-90%, 5%-90%, and 5%-90%, respectively, the preferred proportions are in the range of 10%-50%, 5%-50%, and 12.5%-60%, respectively.

6. The formulation of any one of claims 1 to 5, wherein the formulation enhances the absorption of said pharmaceutical or nutraceutical component in its original form into blood.

7. The formulation of any one of claims 1 to 5, wherein the formulation alters an absorption site of said pharmaceutical or nutraceutical component, and enhances absorption of said pharmaceutical or nutraceutical component in the stomach.

8. The formulation of any one of claims 1 to 5, wherein the formulation enhances oral absorption rate and bioavailability of the pharmaceutical or nutraceutical.

9. The formulation of claim 1 provided as a dosage form comprising a liquid mixture of a pharmaceutical or a nutraceutical, an organic solvent and an acid protectant.

10. The formulation of claim 9, wherein the pharmaceutical component is hydrophilic, lipophilic, or a mixture of hydrophilic and lipophilic.

11. The formulation of claim 9, wherein the pharmaceutical component is selected from one or more of the following: a coumarin, a triterpene saponin, a triterpene sapoginin, a steroid saponin, a flavonoid, a diterpene, a xanthanolide, a quinine, a ginsenoside, ginsenoside Rh2, ginsenoside Rg3, a protopanaxadiol, a protopanaxatriol, a total ginsenoside, an astragaloside, a platycodigenin, a saikosaponin, a dioscin, baicalin, puerarin, genistein, daidzin, a ginkgolide, a bilobalide, bee wax, psoralen, imperatorin, or salviol.

12. The formulation of claim 9, wherein the pharmaceutical component is selected from one or more of the following: a coumarin, a triterpene saponin, a triterpene sapoginin, a steroid saponin, a flavonoid, a diterpene, a xanthanolide, a quinine, a ginsenoside, including ginsenoside Rh2 or ginsenoside Rg3, a protopanaxadiol, a protopanaxatriol, a total ginsenoside, a astragaloside, a platycodigenin, a saikosaponin, a dioscin, and baicalin.

13. The formulation of claim 9, wherein the pharmaceutical component is selected from one or more of the following: ginsenoside Rh2, Rg3, protopanaxadiol, protopanaxatriol, other individual ginsenosides and total ginsenosides.

14. The formulation of claim 9, wherein the pharmaceutical component is selected from one or more of the following: astragalosides, platycodigenins, saikosaponins, dioscin, baicalin.

15. The formulation of claim 9, wherein said organic solvents is selected from one or a mixture of the following: fish oil, safflower oil, refined soybean oil, refined peanut oil, evening primrose oil, milk thistle oil, grape seed oil, sunflower oil, seabuckthorn oil and other animal or plant oil, ethanol, propylene glycol, glycerol, polyethlene glycol, ethyl acetate, and propylene carbonate.

16. The formulation of claim 9, wherein said acid protectant is selected from one or more of the following: Labrafil M 1944 CS, Tween 20, Tween 21, Tween 40, Tween 60, Tween 80, Tween 81, Brij, Spans (Sorbitan fatty acid ester), polyethlene glycol 200-polyethlene glycol 600, Soybean Phospholipids, lecithin, polyglycides, sucrose ester, polyoxyl hydrogenated vegetable oil (for example, polyoxyl hydrogenated castor oil, Cremophor RH 40), polyoxyethylene sorbitan fatty acid ester (for example, POE(20) sorbitan monolaurate, Tween 20), Nikkol HCO 40, Nikkol HCO 50, Nikkol HCO 60, sodium dodecyl sulphate, magnesium dodecyl sulphate, sodium dodecylsulfonate, sodium tetradecyl sulphate, and glycerol monostearate.

17. The formulation of claim 9, wherein said acid protectant is ethanol and polyoxyl hydrogenated vegetable oil product (polyoxyl hydrogenated castor oil).

18. The formulation of claim 9, wherein the weight proportions of pharmaceutical or nutraceutical agent, ethanol and polyoxyl hydrogenated castor oil with respect to the formulation are in the range of 5%-90%, 5%-90%, and 5%-90%, respectively, and the optimal proportions are in the range of 10%-50%, 5%-50%, and 12.5%-60%, respectively.

19. The formulation of any one of claims 9 to 18, wherein the formulation enhances the absorption of pharmaceuticals or nutraceuticals into blood.

20. The formulation of any one of claims 9 to 18, wherein the formulation changes the absorption site, and thus enhances the absorption of the pharmaceutical and nutraceutical component in the stomach.

21. The formulation of any one of claims 9 to 18, wherein the formulation enhances the oral absorption rate and bioavailability of the pharmaceutical or nutraceutical component.

22. The formulation of any one of claims 1 to 18, wherein the dosage form can be oral solution, oral softgel capsule or oral liquid-filled hard gelatin capsule, as well as other well-known applicable dosage forms.

23. A method employed to facilitate the absorption of active drug component in the stomach and therefore to enhance overall bioavailability of the active drug component, wherein: the oral dosage form comprises pharmaceuticals or nutraceuticals which are dissolved in a composition formulation of organic solvent; the primary absorption site is changed from the conventional intestinal tract to stomach; the increased solubility makes possible the direct absorption of intact drugs other than their metabolites, therefore, improving the overall drug bioavailability; comprising the following detailed steps:
step 1: determine the solubility of pharmaceuticals or nutraceuticals in
organic solvents; the solubility is assayed by a well-recognized method or obtained from chemical databases; their optimal proportion will be determined according to drug solubility data;
step 2: (a) completely dissolve the test pharmaceutical or nutraceutical substance into organic solvents of various grades; one or more organic solvents is selected from the followings: fish oil, safflower oil, refined soybean oil, refined peanut oil, evening primrose oil, milk thistle oil, grape seed oil, sunflower oil, seabuckthorn oil and other animal or vegetable oil, ethanol, propylene glycol, glycerol, polyethlene glycol, ethyl acetate, propylene carbonate; the composition solution is called "intermediate solution A"; (b) determine the proportion of protectants in the "intermediate solution A"; one or more protectants is selected from the followings: Labrafil M 1944 CS, Tween 20, Tween 21, Tween 40, Tween 60, Tween 80, Tween 81, Brij, Spans(Sorbitan fatty acid ester), polyethlene glycol 200-polyethlene glycol 600, Soybean Phospholipids, lecithin, polyglycides, sucrose ester, polyoxyl hydrogenated vegetable oil (for example, polyoxyl hydrogenated castor oil, Cremophor RH 40), polyoxyethylene sorbitan fatty acid ester (for example, POE(20) sorbitan monolaurate, Tween 20), Nikkol HCO 40, Nikkol HCO 50, Nikkol HCO 60, sodium dodecyl sulphate, magnesium dodecyl sulphate, sodium dodecylsulfonate, sodium tetradecyl sulphate, and glycerol monostearate; following the well-recognized experimental protocols, the organic solution of pharmaceuticals or nutraceuticals with acid protectant is added into pre-prepared artificial gastric juice and intestinal juice for stability test; using complete dissolution and no precipitation as criteria, a relative proportion of protectant is determined; the acquired relative proportions of pharmaceutical(s) or nutraceutical(s), above-mentioned organic solvent(s), and protectant(s) will be called the proprietary composition formulation of this invention;
step 3: in accordance to pharmaceutical and food industries good manufacture practices, the composition formulation of pharmaceuticals or nutraceuticals is materialized into oral dosage forms including oral solution, softgel capsule, liquid-filled gelatin capsule etc; other food flavorings, edible pharmaceutical adjuvants, or food additives are applicable as well.

24. The method of claim 23, wherein the method is used for the manufacture of drugs, health products, nutraceutical products or functional foods in oral dosage form.

25. The method of claim 23, wherein the method is applied to the manufacture of oral dosage form of antiulcer drugs, analgesics, antihypertensive drugs, cardiovascular drugs, antibiotics, antipsychotic drugs, anticancer drugs, antimuscarinic, diuretics, antimigraine, antiviral drugs, anti-inflammatory agents, sedatives, endocrine modulators, antidiabetic drugs, antidepressant drugs, antihistamines, parasiticides, antiepileptic drugs, antihyperlipidemic drugs etc., as well as health products, nutraceutical products, health foods, functional foods, etc.
